# EUROPÄISCHE PATENTSCHRIFT

(11) **EP 1 812 002 B1**
(45) Veröffentlichungstag und Bekanntmachung des Hinweises auf die Patenterteilung: **05.10.2011**
(21) Anmeldenummer: 05800007.6
(22) Anmeldetag: 28.10.2005
(51) Int. Cl.: A61K 31/496, A61P 13/00, A61P 33/00

(54) **BEHANDLUNG VON MASTITIS**
TREATMENT OF MASTITIS
TRAITEMENT DE MASTITES

(30) Priorität: 12.11.2004 DE 102004054873
(43) Veröffentlichungstag der Anmeldung: 01.08.2007
(73) Patentinhaber: Bayer Animal Health GmbH, 51368 Leverkusen (DE)
(72) Erfinder: PIRRO, Franz, 40764 Langenfeld (DE); FRAATZ, Kristine, 51399 Burscheid (DE); FROYMAN, Robrecht, 40789 Monheim (DE)
(86) Internationale Anmeldenummer: PCT/EP2005/011553
(87) Internationale Veröffentlichungsnummer: WO 2006/050826

(56) Entgegenhaltungen:
- RANTALA M ET AL: "Efficacy and pharmacokinetics of enrofloxacin and flunixin meglumine for treatment of cows with experimentally induced Escherichia coli mastitis" JOURNAL OF VETERINARY PHARMACOLOGY AND THERAPEUTICS, Bd. 25, Nr. 4, August 2002 (2002-08), Seiten 251-258, XP008064401 ISSN: 0140-7783
- KUTILA T ET AL: "The efficacy of bovine lactoferrin in the treatment of cows with experimentally induced Escherichia coli mastitis" JOURNAL OF VETERINARY PHARMACOLOGY AND THERAPEUTICS, Bd. 27, Nr. 4, August 2004 (2004-08), Seiten 197-202, XP008064402 ISSN: 0140-7783
- MUKHERJEE REENA ET AL: "Immunomodulatory and therapeutic potential of enrofloxacin in bovine sub clinical mastitis." ASIAN-AUSTRALASIAN JOURNAL OF ANIMAL SCIENCES, Bd. 16, Nr. 6, Juni 2003 (2003-06), Seiten 889-893, XP008064409 ISSN: 1011-2367
- PIRRO F: "Susceptibility of European bovine mastitis pathogens to enrofloxacin (active of BaytrilTM) and other antiinfectives." JOURNAL OF VETERINARY PHARMACOLOGY AND THERAPEUTICS, Bd. 26, Nr. Supplement 1, August 2003 (2003-08), Seite 237, XP008064408 & PROCEEDINGS OF THE 9TH INTERNATIONAL CONGRESS OF THE EUROPEAN ASSOCIATION FOR VETERINARY PHARMACOLOG; LISBON, PORTUGAL; JULY 13-18, 2003 ISSN: 0140-7783
- LEHTOLAINEN T ET AL: "In vitro antimicrobial susceptibility of Escherichia coli isolates from clinical bovine mastitis in Finland and Israel." JOURNAL OF DAIRY SCIENCE, Bd. 86, Nr. 12, Dezember 2003 (2003-12), Seiten 3927-3932, XP008064476 ISSN: 0022-0302
- MELLGREN CATARINA ET AL: "Optical immunobiosensor assay for determining enrofloxacin and ciprofloxacin in bovine milk" JOURNAL OF AOAC INTERNATIONAL, Bd. 81, Nr. 2, März 1998 (1998-03), Seiten 394-397, XP008064487 ISSN: 1060-3271
- JAYAKUMAR K ET AL: "Disposition of ciprofloxacin in milk after intravenous administration in lactating cows" INDIAN VETERINARY JOURNAL, Bd. 77, Nr. 80644856, Juni 2000 (2000-06), Seiten 495-498, XP008064485 ISSN: 0019-6479
- PAUL W MANOHAR ET AL: "Antibiogram typing of Staphylococcus aureus of bovine mastitis origin" INDIAN JOURNAL OF ANIMAL SCIENCES, Bd. 65, Nr. 3, 1995, Seiten 256-260, XP008064488 ISSN: 0367-8318
- PALMERO DOMINGO J ET AL: "Mastitis due to Mycobacterium fortuitum in an HIV negative patient" MEDICINA (BUENOS AIRES), Bd. 64, Nr. 6, 2004, Seiten 529-532, XP008064486 ISSN: 0025-7680
- SCHROEDER ANKE ET AL: "Susceptibility of mastitis pathogens in northern Germany" BERLINER UND MUNCHENER TIERARZTLICHE WOCHENSCHRIFT, Bd. 118, Nr. 9-10, Oktober 2005 (2005-10), Seiten 393-398, XP008064406 ISSN: 0005-9366
- SALUJA P S ET AL: "Efficacy of enrofloxacin alone or along with immunomodulators therapy for mastitis control in dry cows" INDIAN VETERINARY JOURNAL, Bd. 82, Nr. 3, März 2005 (2005-03), Seiten 264-266, XP008064407 ISSN: 0019-6479
- DAGMAR HOEBEN ET EL.: "treatment of acute E. coli mastitis ijn cows...", JOURNAL OF DAIRY RESEARCH, vol. 67, 2000, pages 485-502,
- DOSOGNE ET AL.: "effects of enrofloxacin treatment on plasma...", INFLAMM. RESEARCH, vol. 51, 2002, pages 201-205,

## Beschreibung

Die Erfindung betrifft die vereinfachte Behandlung der Mastitis mit Enrofloxacin bei Kühen.

Der Wirkstoff Enrofloxacin wird seit Jahren in vielen Ländern zur Behandlung von bakteriell bedingten Infektionserkrankungen bei Tieren erfolgreich angewandt (Baytril®). Die klassischen Einsatzgebiete umfassen in erster Linie Respirations- und enterische Erkrankungen, aber auch Haut-, Harnwegs-, Gesäuge- oder Gelenkinfektionen werden erfolgreich behandelt. Das übliche Behandlungsschema sieht dabei mehrmalig Applikationen über drei bis fünf Tage vor. Versuche zur Verkürzung der Behandlungsdauer bei Beibehaltung der Dosierungshöhe führten in der Vergangenheit zum Verlust der angestrebten therapeutischen Wirksamkeit.

US 5 756 506 betrifft die Behandlung von Infektionen mittels einmaliger Applikation von Fluorchinolonen, wie z.B. Enrofloxacin, allerdings wird hier eine deutlich erhöhte Dosis eingesetzt.

Überraschenderweise hat sich nun gezeigt, dass parenteral intravenös verabreichtes Enrofloxacin bei der Behandlung von Mastitiden (Euterentzündungen) eine unerwartet gute Wirkung hat, sodass die Anzahl der Applikationen verringert und die Behandlung damit vereinfacht werden kann.

Die Erfindung betrifft daher die Verwendung von Enrofloxacin zur Herstellung von Arzneimitteln für die parenterale Behandlung von bakteriell bedingten Mastitiden bei kühen durch maximal zwei intravenöse Applikationen.

Bei der Behandlung von bakteriell bedingten Mastitiden, wird daher so verfahren, dass man dem betreffenden Tier Enrofloxacin höchstens zweimal parenteral intravenös appliziert.

Ohne dass hierdurch die Erfindung eingeschränkt werden soll, kann diese überraschende Erkenntnis durch folgende Untersuchungsergebnisse erklärt werden:

Aus Untersuchungen zur Serumkinetik war bereits bekannt, dass nach Applikation das Enrofloxacin zu einem kleinen Teil zu Ciprofloxacin metabolisiert wird. Allerdings ist üblicherweise die Wirkung des Enrofloxacins auch tatsächlich im Wesentlichen auf dieses Molekül und nicht auf den Metaboliten Ciprofloxacin zurückzuführen. Bei Untersuchungen der antibakteriell wirksamen Stoffe in der Kuhmilch nach parenteraler Applikation von Enrofloxacin entdeckten wir eine hohe antibakterielle Aktivität (Anreichern von active im Verhältnis zur Serumkonzentration) bei überraschend hohem Anteil von Ciprofloxacin (in der Größenordnung von 90 %) und einen

### Dokumente:

KUTILA T ET AL: "The efficacy of bovine lactoferrin in the treatment of cows with experimentally induced Escherichia coli mastitis" JOURNAL OF VETERINARY PHARMACOLOGY AND THERAPEUTICS, Bd. 27, Nr. 4, August 2004, Seiten 197-202
MUKHERJEE REENA ET AL: "Immunomodulatory and therapeutic potential of enrofloxacin in bovine sub clinical mastitis." ASIAN-AUSTRALASIAN JOURNAL OF ANIMAL SCIENCES, Bd. 16, Nr. 6, Juni 2003 (2003-06), Seiten 889-893
und
MELLGREN CATARINA ET AL: "Optical immunobiosensor assay for determining enrofloxacin and ciprofloxacin in bovine milk" JOURNAL OF AOAC INTERNATIONAL, Bd. 81, Nr. 2, März 1998 (1998-03), Seiten 394-397
offenbaren die erfolgreiche dreimalige Applikation von Enrofloxacin zur Behandlung von Mastitis: 5mg/kg i.v. nach 12 Stunden, und dann s.c. zweimal, 24 Stunden voneinander (nach 36 und 60 Stunden). Die Verabreichungsweise von Enrofloxacin ist daher einmal pro Tag für drei Tagen und nicht mit zwei Applikationen, wie beansprucht.

### Die Dokumente :

Journal of Dovey Research, 2000, 67,485-502 und
Inflamm res. 51 (2002),201-205
offenbaren die Verwendung von Enrofloxacin Zur Behandlung von Mestitis, durch maximal zwei Applikationen, eine intravenös und eine subkutan.

überraschend geringen Anteil von Enrofloxacin (in der Größenordnung von 10 %) in der Milch; dies ist etwa eine Verhältnisumkehr dessen was erwartet wurde. In vitro Wirksamkeitsvergleiche zeigen, dass Ciprofloxacin bei Bakterienarten, die bei Mastitiden als Erreger eine wesentliche Rolle spielen, deutlich wirkstärker ist als Enrofloxacin.

Ciprofloxacin kann daher ebenfalls zur Herstellung von Arzneimitteln zur Behandlung von Mastitis verwendet werden.

Enrofloxacin ist eine Fluorchinoloncarbonsäure mit der systematischen Bezeichnung 1-cylopropyl-7-(4-ethyl-1-piperazinyl)-6-fluoro-1,4-dihydro-1,4-dihydro-4-oxo-3-chinoloncarbonsäure:

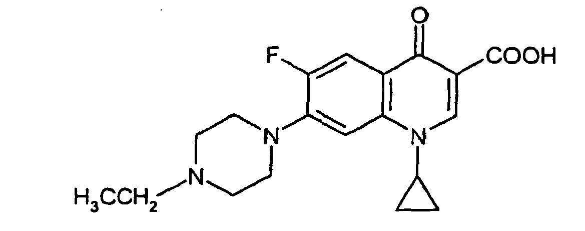

Ciprofloxacin hat die systematische Bezeichnung 1-Cyclopropyl-7-(1-piperazinyl)-6-fluoro-1,4-dihydro-4-oxo-3-chinoloncarbonsäure:

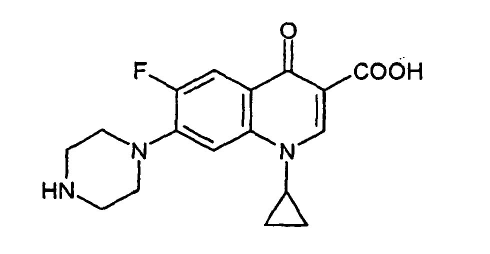

Die Wirkstoffe können in Form ihrer pharmazeutisch annehmbaren Salze eingesetzt werden, und zwar in Form von Salzen mit anorganischen Säuren, wie z.B. Chlorwasserstoffsäure, Bromwasserstoffsäure, Iodwasserstoffsäure, Schwefelsäure, Phosphorsäure oder organischen Säuren wie z.B. Ameisensäure, Essigsäure, Propionsäure, Milchsäure, Maleinsäure, Fumarsäure, Citronensäure, Ascorbinsäure, Bernsteinsäure, Glutarsäure, Weinsäure, Polyhydroxycarbonsäuren wie z.B. Glukonsäure, Galacturonsäure, Gluconsäure, Aminosäuren wie z.B. Glutaminsäure und Asparaginsäure, Sulfonsäuren wie z. B. Methansulfonsäure und Ethansulfonsäure. Für die Salzbildung geeignete Basen sind z.B. anorganische Basen wie NaOH, KOH, Ca(OH)₂, Ammoniak und organische Basen wie Amine, z.B. Mono-, Di- und Trialkylamine, substituierte Amine wie z.B. Ethanolamin, cyclische Amine wie z.B. Morpholin oder Piperazin, basische Aminosäuren wie z.B. Arginin, Lysin, Codein oder N-Methylglucamin. Die Wirkstoffe und ihre Herstellung sind z.B. beschrieben in US 4 670 444.

Zubereitungen für die intravenöse Applikation sind ebenfalls im Prinzip bekannt, siehe z.B. US 4 772 605, US 5 998 418 auf die hier ausdrücklich Bezug genommen wird.

Für die parenterale Applikation kommen Emulsionen, Suspensionen und insbesondere Lösungen in Frage.

Bevorzugtes Lösungsmittel ist Wasser, das gegebenenfalls auch im Gemisch mit anderen Lösungsmitteln eingesetzt werden kann. Zu diesen anderen Lösungsmitteln zählen: Alkohole wie ein- oder mehrwertige primäre oder sekundäre oder tertiäre Alkohole (z.B. Ethanol, Butanol, Benzylalkohol, Glykol, Propylenglykol, Triethylenglykol, Polyethylenglykol, Glycerin, Propylenglykol) sowie N-Methylpyrrolidon.

Es sind jedoch auch Zubereitungen auf Ölbasis denkbar, hierbei handelt es sich üblicherweise um Suspensionen. Die Wirkstoffe liegen in den erfindungsgemäßen Zubereitungen im Allgemeinen in Konzentrationen von 0,1 bis 30 Gew.%, bevorzugt 0,5 bis 20 Gew.%, besonders bevorzugt 1 bis 10 Gew.-% vor.

Die Verwendung von hochreinen Chinoloncarbonsäuren bei der Herstellung von parenteral verabreichbaren Lösungen wird in EP-A-287 926 beschrieben, auf dieses Dokument wird hiermit ausdrücklich Bezug genommen.

Saure Formulierungen können eingesetzt werden, bevorzugte pH-Werte liegen im Bereich pH 3 bis 6,5, besonders bevorzugt 3 bis 5. Als Säuren können prinzipiell die weiter oben für die Salzbildung genannten eingesetzt werden, bevorzugte Beispiele sind Milchsäure und Gluconolacton. Lösungen milchsaurer Salze von Chinoloncarbonsäuren, insbesondere Ciprofloxacin, die sich für Injektionszwecke eignen, werden in EP-A-138 018 beschrieben; weitere saure Infusionslösungen des Ciprofloxacins offenbart EP-A-219 784; saure Injektionslösungen für Enrofloxacin werden in US 5 998 418 beschrieben; auf die drei vorstehend genannten Dokumente wird hiermit ausdrücklich Bezug genommen.

Bevorzugt sind basische Formulierungen, die überäquimolare Mengen an Basen enthalten, solche Zubereitungen haben einen pH von 8 bis 12,5 bevorzugt 9 bis 12, besonders bevorzugt 9,5 bis 11,5. Als Basen kommen z. B. die oben im Zusammenhang mit den Salzen genannten Basen in Frage, vorzugsweise die Alkalihydroxide wie NaOH und insbesondere KOH. Weiterhin ist als Base besonders bevorzugt das Arginin. Solche Formulierungen sind z.B näher beschrieben in US 4 772 605, auf dieses Dokument wird hiermit ausdrücklich Bezug genommen.

Die Arzneimittelzubereitungen können weiterhin übliche Hilfsstoffe enthalten; dies sind nichttoxische pharmazeutische Stoffe wie Verdünnungsmittel, Verdickungsmittel Resorptionsbeschleuniger, Resorptionshemmer, Kristallisationsverzögerer, Komplexiermittel, Lichtschutzmittel, Antioxidantien, Konservierungsmittel. Beispielhaft seien genannt: Als Verdickungsmittel Methylcellulose, Hydroxyethylcellulose, Hydroypropylcellulose, Natriumcarboxymethylcellulose, Polyvinylpyrrolidon, Gelatine; als Konservierungsmittel p-Hydroxybenzoesäureester, Phenole, Chlorbutanol, Benzylalkohol, Ethanol, Butanol, 1,3-Butandiol, Chlorhexidin-Salze, Benzoesäure und Salze, Sorbinsäure; als Antioxidatien Ascorbinsäure, L-Cystein, Thiodipropionsäure, Thiomilchsäure, Monthioglycerin, Propylgallat, Natrium-metadisulfit oder Natriumsulfit; als Komplexiermittel Natriumsalze der Ethylendiamintetraessigsäure, Phosphate, Acetate, Citrate; als Kristallisationsverzögerer Polyvinylpyrrolidon. Gegebenenfalls können Lokalanästhetika wie z. B. Procainhydrochlorid oder Lidocainhydrochlorid zugesetzt werden. Die Konzentration der gegebenenfalls eingesetzten Hilfsstoffe variiert stark und kann in üblichen Formulierungen im Bereich von 0,1 bis 30 Gew.% für die Gesamtmenge der Hilfsstoffe liegen.

Zur Einstellung isotoner Verhältnisse können z. B. Kochsalz, Glucose, Fructose, Glycerin, Sorbit, Mannit, Saccharose, Xylit oder Mischungen dieser Stoffe in geeigneter Menge zugegeben werden.

Prinzipiell können erfindungsgemäß bakteriell bedingte, insbesondere coliforme Mastitiden bei allen Säugetieren behandelt werden. Von besonderer Bedeutung ist jedoch die Behandlung von milchgebenden Nutztieren, als bevorzugte Beispiele seien genannt: Schaf, Ziege und insbesondere Kühe. Als Erreger in diesem Zusammenhang sind insbesondere die Folgenden zu nennen: E. coli, Klebsiella spp, Enterobacter spp., Salmonella spp., Citrobacter spp., Serratia spp., Shigella spp., Edwardsiella spp., Hafnia spp., Morganella spp., Providencia spp., Yersinia spp., Staphylococcus aureus, Staphylococcus spp., Pseudomonas spp., Mycoplasma spp. oder Erwinia spp., und folgende, durch nicht-coliforme Bakterien verursachte Infektionen der Milchdrüse.

Die Verabreichung erfolgt intravenös und zwar üblicherweise durch Injektion .

Bei der Behandlung werden pro Tag üblicherweise 1 bis 10 mg/kg, bevorzugt 2 bis 8 mg/kg, besonders bevorzugt 2,5 bis 7 mg/kg Körpergewicht des Wirkstoffs verabreicht. Vorzugsweise erfolgt die Applikation an zwei aufeinander folgenden Tagen. Üblicherweise ist pro Tag nur eine Applikation notwendig.

Zudem werden häufig vorkommende Misch- und Monoinfektionen oder Mischinfektionen von z.B. E.coli und Staphylococcus oder Mycoplasma gut therapiert.

### Beispiele

### Formulierungsbeispiele

Die Formulierungen der folgenden Beispiele können erfindungsgemäß eingesetzt werden. Die Herstellung ist im Stand der Technik bekannt:

### Beispiel 1

100 ml enthalten:

| | |
|---|---|
| 10,0 g | Enrofloxacin |
| 8,0 g | Gluconolacton |
| 1,40 g | Benzylalkohol |
| 0,1 g | Natriumsulfit |
| 86,7 g | Wasser (für Injektionszwecke) |
| | pH = 3,90 |

### Beispiel 2

100 ml enthalten:

| | |
|---|---|
| 5,0 g | Enrofloxacin |
| 3,0 g | Gluconolacton |
| 1,00 g | Benzylalkohol |
| 0,1 g | Natriumsulfit |
| 93,6 g | Wasser (für Injektionszwecke) pH = 4,40 |

### Beispiel 3

100 ml enthalten:

| | |
|---|---|
| 5,0 g | Enrofloxacin |
| 3,0 g | n-Butanol |
| | KOH bis pH 11 |
| q.s. | Wasser (für Injektionszwecke) |

### Beispiel 4

100 ml enthalten:

| | |
|---|---|
| 10,0 g | Enrofloxacin |
| 3,0 g | n-Butanol |
| | KOH bis pH 11 |
| q.s. | Wasser (für Injektionszwecke) |

### Beispiel 5

100 ml enthalten:

| | |
|---|---|
| 10,0 g | Enrofloxacin |
| 3,0 g | n-Butanol |
| 2,0 g | Benzylalkohol |
| 20,0 g | L-Arginin |
| q.s. | Wasser (für Injektionszwecke) |

### Beispiel 6 (nicht erfindungsgemäβ)

100 ml enthalten:

| | |
|---|---|
| 200 mg | Ciprofloxacin |
| 321,8 mg | Milchsäurelösung |
| 900 mg | NaCl |
| 140 mg | Salzsäure |
| q.s. | Wasser (für Injektionszwecke) |

### Beispiel 7 (nicht erfindungsgemäβ)

100 ml enthalten:

| | |
|---|---|
| 200 mg | Ciprofloxacin |
| 372,5 mg | Milchsäure 10% (g/g) |
| 900 mg | NaCl |
| 10,4 mg | Salzsäure |
| q.s. | Wasser (für Injektionszwecke) pH = 3,7 |

### Beispiel 8 (nicht erfindungsgemäβ)

100 ml enthalten:

| | |
|---|---|
| 100 mg | Ciprofloxacin |
| 320 mg | Milchsäure 10% (g/g) |
| 625 mg | NaCl |
| q.s. | Wasser (für Injektionszwecke) |
| | pH = 4,4 |

### Beispiel 9 (nicht erfindungsgemäβ)

100 ml enthalten:

| | |
|---|---|
| 42,4 mg | Ciprofloxacin-Calciumsalz |
| 644 mg | Milchsäure 2% (g/g) |
| 5,06 mg | Salzsäure |
| 520 mg | Glycerin |
| q.s. | Wasser (für Injektionszwecke) |
| | pH = 4,3 |

### Beispiel 10 (nicht erfindungsgemäβ)

100 ml enthalten:

| | |
|---|---|
| 254,5 mg | Ciprofloxacin x 5 H₂O |
| 1284 mg | Milchsäure 5% (g/g) |
| 3,3 mg | Salzsäure |
| 2500 mg | Glukose |
| q.s. | Wasser (für Injektionszwecke) |
| | pH = 4,2 |

### Beispiel 11 (nicht erfindungsgemäβ)

100 ml enthalten:

| | |
|---|---|
| 233 mg | Ciprofloxacin Kaliumsalz |
| 277 mg | Milchsäure 20 % (g/g) |
| 8,86 ml | 0,1 m Salzsäure |
| 5000 mg | Glukose |
| q.s. | Wasser (für Injektionszwecke) |
| | pH = 4,6 |

### Biologisches Beispiel

### Klinische Studie

In einer klinischen Studie wurde die Wirksamkeit von Enrofloxacin (Baytril^{®} 10 % Injektionslösung, Handelsprodukt) bei der Behandlung von Mastitis mit einem zur Behandlung von Mastititis üblichen Handelsprodukt auf Cefquinom-Basis (Cobactan LC^{®}) verglichen. Enrofloxacin wurde in einer Dosierung von 5 mg/kg Körpergewicht 1 mal täglich an zwei aufeinander folgenden Tagen intravenös verabreicht. Cefquinom wurde alle 12 Stunden nach drei aufeinander folgenden Melkvorgängen in einer Dosis von 75 mg intramammär in das infizierte Euterviertel appliziert.

### Ergebnis:

Die Enrofloxacin-Gruppe zeigte insgesamt nach Behandlung einen besseren Zustand als die mit dem Vergleichsprodukt behandelte Gruppe.

Die Behandlung mit Enrofloxacin wurde von allen Kühen gut vertragen.

Die Studie zeigte, dass das Enrofloxacinprodukt bei der angegebenen Applikation für die Behandlung der akuten coliformen Mastititis von Milchkühen geeignet ist. Bei Bewertung der allgemeinen und lokalen Symptome, der Milchleistung und der bakteriologischen Ergebnisse war Enrofloxacin dem Vergleichsprodukt überlegen.

## Patentansprüche

1. Verwendung von Enrofloxacin zur Herstellung von Arzneimitteln für die parenterale Behandlung von Mastitiden bei Kühen durch maximal zwei intravenöse Applikationen.

2. Verwendung gemäß Anspruch 1, wobei die Applikationen an zwei aufeinander folgenden Tagen erfolgen.

3. Verwendung gemäß einem der vorstehenden Ansprüche, wobei die Applikationen jeweils einmal am Tag erfolgen.

4. Verwendung gemäß einem der vorstehenden Ansprüche, wobei pro Tag 1 bis 10 mg/kg Enrofloxacin verabreicht werden.

## Claims

1. Use of enrofloxacin for producing pharmaceuticals for the parenteral treatment of mastites in cows by means of at most two intravenous administrations.

2. Use according to Claim 1, wherein the administrations are effected on two consecutive days.

3. Use according to either of the preceding claims, wherein the administrations are effected in each case once per day.

4. Use according to any of the preceding claims, wherein from 1 to 10 mg/kg of enrofloxacin is administered per day.

## Revendications

1. Utilisation d'enrofloxacine pour la préparation de médicaments destinés au traitement parentéral de mastites chez la vache, au maximum par deux administrations intraveineuses.

2. Utilisation selon la revendication 1, les administrations s'effectuant deux jours consécutifs.

3. Utilisation selon l'une des revendications précédentes, chacune des administrations s'effectuant une fois par jour.

4. Utilisation selon l'une des revendications précédentes, pour laquelle on administre par jour 1 à 10 mg/kg d'enrofloxacine.
